# EUROPEAN PATENT APPLICATION

(11) **EP 2 047 796 A1**
(43) Date of publication of application: **15.04.2009**
(21) Application number: 08016566.5
(22) Date of filing: 19.09.2008
(51) Int. Cl.: A61B 5/053

(54) **Bioelectrical impedance measuring apparatus and body composition determining apparatus**

(30) Priority: 09.10.2007 JP 2007263170
(71) Applicant: TANITA CORPORATION, Tokyo 174-8630 (JP)
(72) Inventor: Kasahara, Yasuhiro, Tokyo 174-8630 (JP); Sakai, Yoshio, Tokyo 174-8630 (JP)
(74) Representative: Grünecker, Kinkeldey, Stockmair & Schwanhäusser Anwaltssozietät

(57) **Abstract**

A bioelectrical impedance measuring apparatus includes a measuring unit for sequentially measuring a bioelectrical impedance of a living subject. The apparatus may further include a period determiner for determining a fluctuation period of fluctuation in values of the bioelectrical impedance measured by the measuring unit, the fluctuation being caused by breathing of the living subject. A calculator calculates an average of a plurality of the values of the bioelectrical impedance measured by the measuring unit within the fluctuation period determined by the period determiner.

## Description

### BACKGROUND OF THE INVENTION

### FIELD OF THE INVENTION

The present invention relates to measurement of a bioelectrical impedance of a living subject.

### RELATED ART

Methods for measuring a bioelectrical impedance of a human subject have conventionally been proposed. For example, Japanese Patent Application Publication JP-2005-288023-A discloses a method for measuring a bioelectrical impedance of an abdomen of a human subject. In the method, a pair of current supplying electrodes and a pair of voltage measurement electrodes are brought into contact with the abdomen of the human subject, and the bioelectrical impedance is measured on the basis of the potential difference between the voltage measurement electrodes when an alternating current passes between the current supplying electrodes through the abdomen of the human subject.

The ratio of the visceral fat area to the skeletal muscle (abdominal muscles) area in a cross section of the abdomen varies in conjunction with diaphragm motion during breathing of the human subject. For this reason, in the method disclosed in Japanese Patent Application Publication JP-2005-288023-A, the bioelectrical impedance measured during exhalations differs from that measured during inhalations.

### SUMMARY OF THE INVENTION

Accordingly, an object of the present invention is to measure a bioelectrical impedance which is less affected by the breathing of a living subject.

In accordance with one aspect of the invention, there is provided a bioelectrical impedance measuring apparatus including: a measuring unit for sequentially measuring a bioelectrical impedance of a living subject; a period determiner for determining a fluctuation period of fluctuation in values of the bioelectrical impedance measured by the measuring unit, the fluctuation being caused by breathing of the living subject; and a calculator for calculating an average of a plurality of the values of the bioelectrical impedance measured by the measuring unit within the fluctuation period determined by the period determiner. With such a structure, the measured values of the bioelectrical impedance that are within the fluctuation period are used for the calculation by the calculator, and an accurate bioelectrical impedance can be obtained which is less affected by the breathing of the living subject.

In a preferred embodiment, the measuring unit starts measuring the bioelectrical impedance at a starting time point, the bioelectrical impedance reaching one of a local maximum and a local minimum at a first time point directly after the starting time point, the bioelectrical impedance reaching the other of the local maximum and the local minimum at a second time point directly after the first time point, and the period determiner determining the fluctuation period as a sum of twice the length of time from the starting time point to the first time point and the length of time from the first time point to the second time point. With such an embodiment, since the impedance values that are measured for a short time immediately after starting the measurements are used for the average calculation by the calculator, the length of time for the measurements of the bioelectrical impedance by the measuring unit can be shortened. However, the method for determining the fluctuation period by the period determiner is not limited to the method described above. For example, in an alternative embodiment, the period determiner determines the fluctuation period as a time interval between two time points at which the bioelectrical impedance reaches two local maximums or minimums.

In an embodiment, the calculator calculates the average of all of the values of the bioelectrical impedance measured by the measuring unit within the fluctuation period determined by the period determiner. With such an embodiment, variation of the average calculated by the calculator can be reduced as compared with an embodiment in which the measured values within the fluctuation period is partially used for calculating the average by the calculator. In an alternative embodiment, the calculator calculates the average of a maximum value and a minimum value of the bioelectrical impedance measured by the measuring unit within the fluctuation period determined by the period determiner. With such an embodiment, since the number of the measured values used for calculating the average by the calculator decreases, the processing load on the calculator can be reduced.

The bioelectrical impedance measuring apparatus may further include a low-pass filter for attenuating high-frequency components of the fluctuation in the bioelectrical impedance measured by the measuring unit, the high-frequency components having frequencies higher than a predetermined cutoff frequency. The period determiner determines the fluctuation period on the basis of the values of the bioelectrical impedance having passed through the low-pass filter, and the calculator calculates the average of the values of the bioelectrical impedance having passed through the low-pass filter. With such an embodiment, since the effects of momentary body motion of the living subject caused by a sneeze, a cough or the like is reduced, a more accurate bioelectrical impedance can be obtained.

The bioelectrical impedance measuring apparatus may further include a high-pass filter for attenuating low-frequency components of the fluctuation in the bioelectrical impedance measured by the measuring unit, the low-frequency components having frequencies lower than a predetermined cutoff frequency. The period determiner determines the fluctuation period on the basis of the values of the bioelectrical impedance having passed through the high-pass filter, and the calculator calculates the average of the value of the bioelectrical impedance having passed through the high-pass filter. With such an embodiment, since influence of the gentle swinging of the living subject is reduced, a more accurate bioelectrical impedance can be obtained.

In a preferred embodiment, the period determiner includes a candidate period determiner for sequentially determining candidate periods of the fluctuation in the values of the bioelectrical impedance measured by the measuring unit, a steady period detector for detecting a series of the candidate periods each having a time length within a predetermined range, and a second period determiner for determining the fluctuation period on the basis of the series of the candidate periods detected by the steady period detector. With such an embodiment, since the values that are measured after the fluctuation becomes substantially periodic are used for the calculation by the calculator, a more accurate bioelectrical impedance can be obtained.

In accordance with another aspect of the invention, there is provided a body composition index determining apparatus including: a bioelectrical impedance measuring apparatus according to above-described embodiments of the invention; and an index calculator for calculating a body composition index of the living subject on the basis of the bioelectrical impedance measured by the bioelectrical impedance measuring apparatus.

### BRIEF DESCRIPTION OF THE DRAWINGS

With reference to the accompanying drawings, various embodiments of the present invention will be described hereinafter. In the drawings:
Fig. 1 is a block diagram showing a body composition determining apparatus according to a first embodiment of the invention;
Fig. 2 is a graph showing fluctuation of bioelectrical impedance caused by breathing of a living subject;
Fig. 3A and Fig. 3B are schematic diagrams showing change in form of elements in a cross section of the abdomen of a living subject;
Fig. 4 is a schematic diagram for an explanation of the function of a period determiner of the body composition determining apparatus in Fig. 1;
Fig. 5 is a block diagram showing a body composition determining apparatus according to a second embodiment of the invention;
Fig. 6 is a block diagram showing a period determiner in a third embodiment of the invention;
Fig. 7 is a schematic diagram for an explanation of the function of the period determiner in the third embodiment; and
Fig. 8 is a schematic diagram for an explanation of a function of a period determiner according to a modification of the first or second embodiment.

### DETAILED DESCRIPTION OF PREFERRED EMBODIMENTS

### FIRST EMBODIMENT

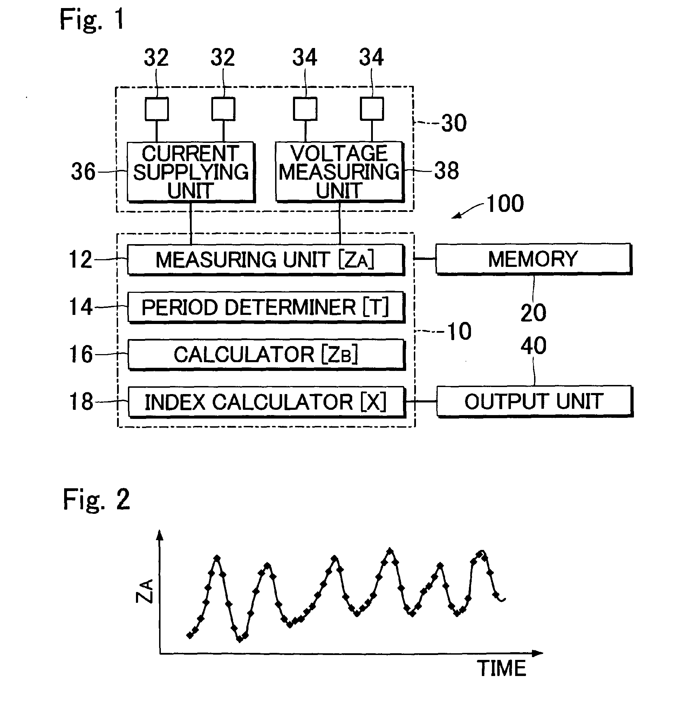

Fig. 1 is a block diagram of a body composition determining apparatus according to a first embodiment of the present invention. The body composition determining apparatus 100 is a device for measuring bioelectrical impedance of the abdomen of a human subject and determining and outputting indexes X regarding body composition (body composition indexes X) on the basis of the measured bioelectrical impedance. As shown in Fig. 1, the body composition determining apparatus 100 includes a controller 10, a memory 20, a measurement electrode part 30, and an output unit 40.

The controller 10 is a processor for controlling the entirety of the body composition determining apparatus 100. The controller 10 functions as a plurality of elements (a measuring unit 12, a period determiner 14, a calculator 16, and an index calculator 18) by executing a program. The memory 20 (e.g., a semiconductor memory) stores the program executed by the controller 10 and various data items used by the controller 10.

The measurement electrode part 30 is used for measuring the bioelectrical impedance of a human subject. As shown in Fig. 1, the measurement electrode part 30 includes a pair of current supplying electrodes 32, a pair of voltage measurement electrodes 34, a current supplying unit 36, and a voltage measuring unit 38.

The current supplying electrodes 32 are brought into contact with various areas of the abdomen of a human subject. The voltage measurement electrodes 34 are brought into contact with various areas of the abdomen of the human subject, the areas being between the current supplying electrodes 32.

The current supplying unit 36 supplies an alternating current (measurement current) having a predetermined frequency between the current supplying electrodes 32 so that the measurement current passes through the abdomen of the human subject. The voltage measuring unit 38 detects a potential difference (measurement voltage) between the voltage measurement electrodes 34 while the measurement current is supplied to the current supplying electrodes 32.

The measuring unit 12 of the controller 10 sequentially measures the bioelectrical impedance of the abdomen of the human subject by using the measurement electrode part 30 and stores a time series (history) of the values Z_{A} of the bioelectrical impedance measured by the measuring unit 12 in the memory 20. More specifically, when an instruction to start measuring is given to an input device (not shown) from a user, the measuring unit 12 directs the current supplying unit 36 to generate the measurement current. The measuring unit 12 then calculates the bioelectrical impedance of the abdomen of the human subject sequentially many times on the basis of the correlation between the measurement current and the measurement voltage detected by the voltage measuring unit 38 while the measurement current passes between the current supplying electrodes 32 through the abdomen of the human subject.

The measuring unit 12 in this embodiment calculates the values Z_{A} of the bioelectrical impedance at regular intervals. A length of time between any two time points corresponds to the number of the measurements (i.e., samplings) of the values Z_{A} between the two time points by the measuring unit 12. As will be described later, the period determiner 14 measures time, but it may count the number of the measurements of the values Z_{A} instead of the measurement of time. In an alternative embodiment, the measuring unit 12 may measure the values Z_{A} at irregular intervals.

Fig. 2 is a graph showing fluctuation of values Z_{A} of the bioelectrical impedance. As shown in Fig. 2, the values Z_{A} of the bioelectrical impedance periodically fluctuate in synchrony with the breathing of the human subject. More specifically, the fluctuation of the values Z_{A} is approximately a sine wave having a frequency from 0.2 Hz to 0.33 Hz (in three to five second periods). A reason for the fluctuation of the values Z_{A} according to breathing is described below.

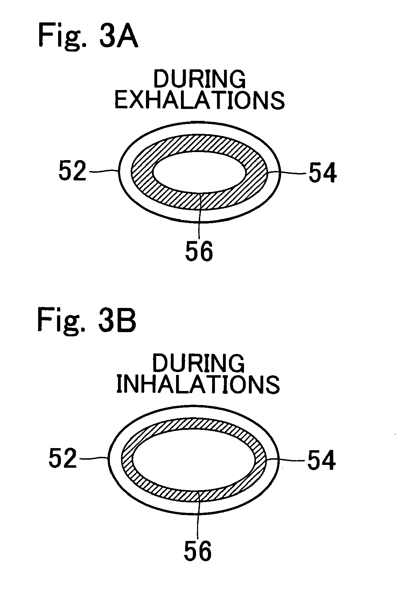

Fig. 3A and Fig. 3B are schematic diagrams showing the change in the configuration of elements (subcutaneous fat 52, abdominal muscles 54, and visceral fat 56) in a cross section of the abdomen according to breathing of the human subject. Fig. 3A shows the cross section of the abdomen during exhalations. Fig. 3B shows the cross section of the abdomen during inhalations.

As shown in Fig. 3B, when the human subject inhales air, the area of visceral fat 56 increases in conjunction with diaphragm motion as compared with that area during exhalations (Fig. 3A), and the thickness of the abdominal muscles 54 decreases with extension caused by the visceral fat 56 inside of the abdominal muscles 54. Since the abdominal muscles 54 has higher conductivity than the visceral fat 56 and the subcutaneous fat 52, the bioelectrical impedance measured during inhalations is higher than that measured during exhalations. For the reason described above, the measured value Z_{A} of the bioelectrical impedance periodically fluctuates according to breathing of the human subject.

Each time interval of the measurements of the value Z_{A} (i.e., the period of the measurement of the bioelectrical impedance) by the measuring unit 12 is set according to an average period (an average frequency) of breathing of human beings so that the fluctuation of the bioelectrical impedance can be determined from the time series (history) of the values Z_{A}. More specifically, it is preferable that the frequency of the measurements of the bioelectrical impedance is set to more than double the average frequency of breathing. Since the average frequency of breathing is 0.2 Hz to 0.33 Hz (in three to five second periods), the frequency of the measurements of the bioelectrical impedance may be set to 0.4 Hz to 0.66 Hz (in 1.5 to 2.5 second periods). In this embodiment, in order to specify the fluctuation in the values Z_{A} with high resolution, the measuring unit 12 sequentially measures the values Z_{A} at a frequency of 5 Hz (in 0.2 second periods).

Furthermore, the length of time during which the measuring unit 12 performs the measurements of the bioelectrical impedance is set so as to be longer than the average period of breathing. Since the average period of breathing is three seconds to five seconds, the measuring unit 12 sequentially measures the values Z_{A} for more than ten seconds after starting the measurement of the bioelectrical impedance (e.g., after the instruction to start measuring).

The period determiner 14 in Fig. 1 determines a period T of the fluctuation in the measured values Z_{A} caused in conjunction with breathing of the human subject. The period (fluctuation period) T corresponds to the period of breathing of the human subject. The period determiner 14 in this embodiment determines the fluctuation period T on the basis of the time series of the values Z_{A} stored in the memory 20. The method described below is, for example, employed for determining the fluctuation period T, but is not limited thereto. However, it is not intended to limit the present invention to this embodiment.

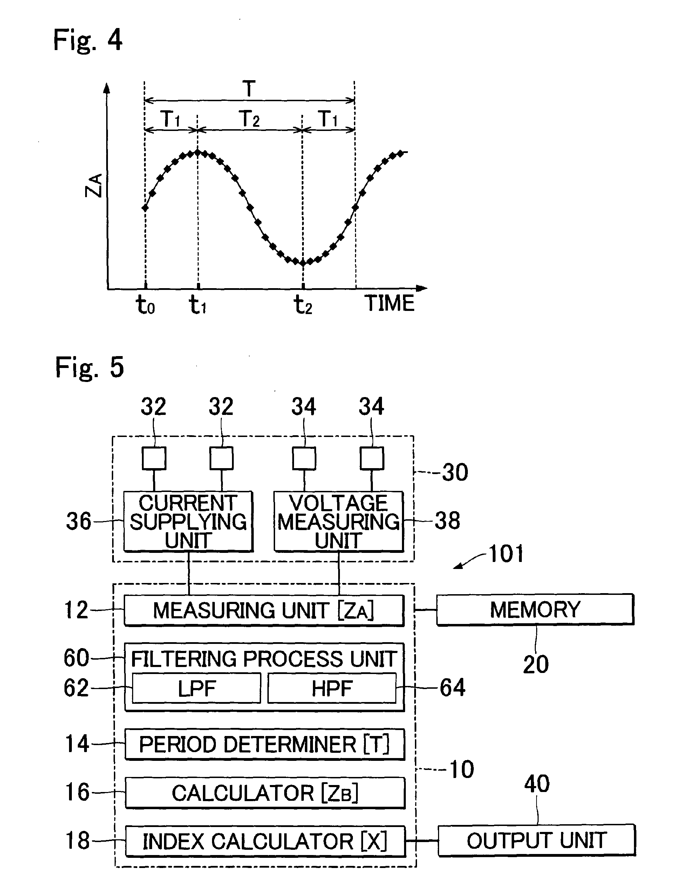

As shown in Fig. 4, the period determiner 14 determines a time length T₁ and a time length T₂. The time length T₁ ranges from a starting time point to to a first time point t₁. The starting time point t₀ is a point at which the value Z_{A} is calculated for the first time (the measurement of the bioelectrical impedance is started). The first time point t₁ is a point at which the bioelectrical impedance measured by the measuring unit 12 reaches an extremum value (one of local maximum and local minimum) directly after the starting time point to. The time length T₂ ranges from the first time point t₁ to a second time point t₂. The second time point t₂ is a point at which the bioelectrical impedance measured by the measuring unit 12 reaches the next extremum value (the other of local maximum and local minimum) directly after the first time point t₁. The period determiner 14 then calculates the fluctuation period T as a sum of twice the time length T₁ and the time length T_{2.}

The calculator 16 in Fig. 1 calculates a determinate value Z_{B} of the bioelectrical impedance on the basis of the measured values Z_{A} within the fluctuation period T determined by the period determiner 14. More specifically, the calculator 16 calculates the determinate value Z_{B} as an average (arithmetic mean or geometric mean) of all of the measured values Z_{A} within the fluctuation period T.

The index calculator 18 in Fig. 1 calculates the body composition indexes X on the basis of the determinate value Z_{B} of the bioelectrical impedance calculated by the calculator 16. The body composition indexes X include, for example, but are not limited to, fat ratio of the entire body, fat ratio of the trunk portion, total fat area of the abdomen, subcutaneous fat area of the abdomen, visceral fat area, subcutaneous fat thickness, and abdominal muscle thickness. For the calculation of each of the body composition indexes X, a regression formula is used that expresses a correlation between the bioelectrical impedance of the abdomen and measured values of the body composition index X. In a preferred embodiment, another index (e.g., height, weight, age, sex, BMI (body mass index)) may be used in conjunction with the bioelectrical impedance for calculating the body composition indexes X.

The output unit 40 outputs the body composition indexes X calculated by the index calculator 18. For example, the output unit 40 may be a display that displays the body composition indexes X, a printer that prints out the body composition indexes X, or a speaker that outputs the body composition indexes X by voice.

As described above, in this embodiment, the determinate value Z_{B} of the bioelectrical impedance is calculated on the basis of the measured values Z_{A} within the fluctuation period T. Therefore, more accurate bioelectrical impedances (and therefore more accurate body composition indexes X), in which fluctuations caused by breathing of the human subject are compensated for, can be obtained as compared with the method in which the bioelectrical impedance is measured at only one time.

### SECOND EMBODIMENT

A second embodiment of the present invention will be described below. Elements in the embodiments described hereinafter having a similar function as the elements in the first embodiment are given the same reference number as those used in the first embodiment, and detailed descriptions of these elements will be omitted.

Fig. 5 is a block diagram showing a body composition determining apparatus 101 in this embodiment. As shown in Fig. 5, the body composition determining apparatus 101 includes a filtering process unit 60 in the controller 10 of which other elements are the same as in the first embodiment.

In addition to breathing, the bioelectrical impedance is also affected by momentary body movements of the human subject caused by a sneeze, a cough or the like, and gentle swaying of the human subject. The filtering process unit 60 reduces the effects of body motion of the human subject other than breathing by filtering the time series of the values Z_{A} of the bioelectrical impedance measured by the measuring unit 12.

As shown in Fig. 5, the filtering process unit 60 includes a low-pass filter ("LPF" in Fig. 5) 62 and a high-pass filter ("HPF" in Fig. 5) 64. The low-pass filter 62 reduces the effects of momentary body movements caused by a sneeze, a cough or the like by attenuating high-frequency components of the fluctuation in the measured values Z_{A}. The high-frequency components fall within a frequency band that is higher than a cutoff frequency F₁. The cutoff frequency F₁ is set to a frequency higher than the average frequency of breathing (0.2 Hz to 0.33 Hz) and lower than an experimental standard frequency of fluctuation of bioelectrical impedance caused by momentary body motion. For example, the cutoff frequency F₁ is set to 10 Hz.

The high-pass filter 64 reduces the effects of gentle body swaying by attenuating low-frequency components of fluctuation in the measured values Z_{A}. The low-frequency components fall within a frequency band that is lower than a cutoff frequency F₂. The cutoff frequency F₂ is lower than the cutoff frequency F₁. More specifically, the cutoff frequency F₂ is set to a frequency lower than the average frequency of breathing (0.2 Hz to 0.33 Hz), but higher than an experimental standard frequency of gentle body swaying. For example, the cutoff frequency F₂ is set to 0.05 Hz.

The time series of the measured values Z_{A} having passed through the filtering process unit 60 (the low-pass filter 62 and the high-pass filter 64) are stored in the memory 20. The period determiner 14 determines the fluctuation period T on the basis of the filtered time series of the values Z_{A} in the same manner as that in the first embodiment. The calculator 16 calculates the determinate value Z_{B} on the basis of the filtered time series of the values Z_{A} in the same manner as that in the first embodiment.

As mentioned above, the filtering process unit 60 attenuates the high-frequency components having frequencies that are higher than the cutoff frequency F₁ and the low-frequency components having lower frequencies than the cutoff frequency F₂ of the time series of the values Z_{A}. Therefore, more accurate bioelectrical impedances (and therefore more accurate body composition indexes X) can be obtained as a result of reducing the effects of momentary body movements and gentle body swaying of the human subject, in addition to breathing.

### THIRD EMBODIMENT

A third embodiment of the present invention will be described below.

Breathing of the human subject may be irregular (aperiodic) immediately after starting the measurement of the bioelectrical impedance. In this embodiment, the time series of the values Z_{A} measured after breathing of the human subject becomes substantially regular is used to determine the fluctuation period T and to calculate the determinate value Z_{B} of the bioelectrical impedance.

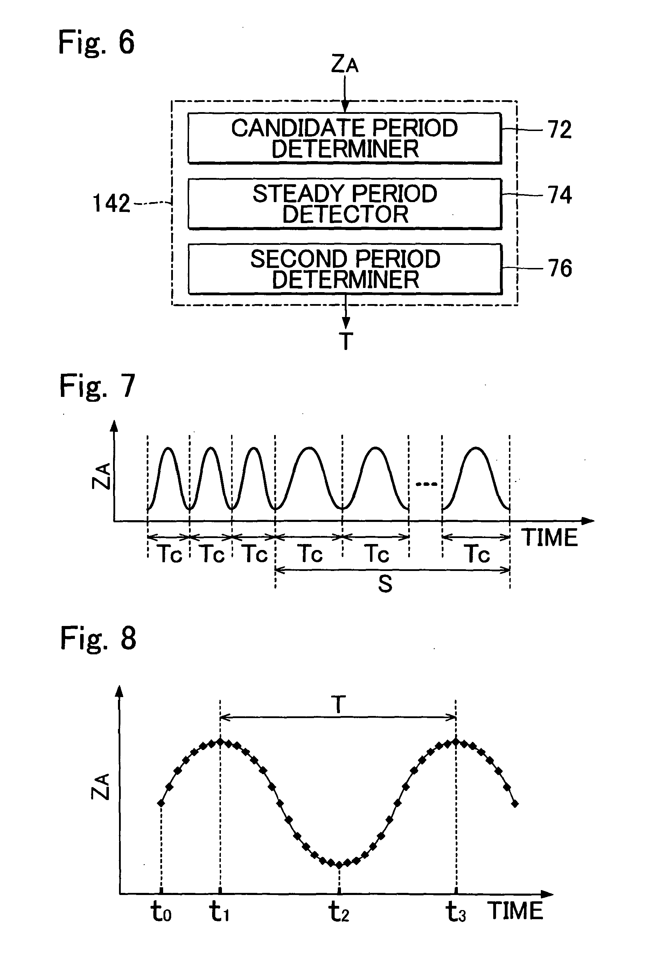

Fig. 6 is a block diagram showing a period determiner 142 of a body composition determining apparatus in this embodiment. In a manner similar to that of the period determiner 14 in the first embodiment, the period determiner 142 determines the fluctuation period T. As shown in Fig. 6, the period determiner 142 includes a candidate period determiner 72, a steady period detector 74, and a second period determiner 76.

As shown in Fig. 7, the candidate period determiner 72 sequentially determines candidate periods Tc of the fluctuation in the values Z_{A} of the bioelectrical impedance measured by the measuring unit 12. The candidate period determiner 72 determines each of the candidate periods Tc, for example, in the same manner as determining the fluctuation period T in the first embodiment, but this is not limited thereto.

The steady period detector 74 in Fig. 6 decides whether or not breathing by the human subject has become stable (i.e., whether or not the time series of the measured values Z_{A} exhibits accurate periodicity). For example, the steady period detector 74 performs the process described below.

The steady period detector 74 decides whether or not each time length of the candidate periods Tc determined by the candidate period determiner 72 is within a predetermined range (reference range). The upper limit and the lower limit of the reference range are stored in the memory 20 in advance. As shown in Fig. 7, the steady period detector 74 then detects a series S of the predetermined number of the candidate periods Tc each having a time length within the reference range. The number of the candidate periods Tc within the series S is, for example, three to five.

The second period determiner 76 in Fig. 6 determines the fluctuation period T on the basis of the series S of the candidate periods Tc detected by the steady period detector 74. For example, the second period determiner 76 selects one of the candidate periods in the series S as the fluctuation period T. In an alternative embodiment, the second period determiner 76 may calculate the fluctuation period T as the average of two or more candidate periods Tc selected from among the series S.

As described above, in this embodiment, the determinate value Z_{B} of the bioelectrical impedance (and therefore the body composition index X) is calculated after breathing by the human subject becomes periodic. Therefore, bioelectrical impedance can be more accurately calculated as compared with the first or second embodiment, in which the values Z_{A} that were measured immediately after the start of the measurements were used for determining the value Z_{B}.

In this embodiment, the steady period detector 74 detects the stability of breathing by the human subject by using the values Z_{A} measured by the measuring unit 12. In an alternative embodiment, the values Z_{A} filtered by the filtering process unit 60 in the second embodiment are used to detect stability of breathing by the human subject.

### MODIFICATIONS

Various modifications may be made to the embodiments described above. Exemplary modifications will be described below. Two or more of the modifications selected from among the modifications below may be combined.
(1) The method for determining the fluctuation period T is not limited to the method described above. For example, the period determiner 14 may determine the fluctuation period T as a time interval between two time points at which the measured values Z_{A} reach local maximums. Alternatively, the period determiner 14 may determine the fluctuation period T as a time interval between two time points at which the measured values Z_{A} reach local minimums.
   More specifically, the period determiner 14 sequentially detects the extremum time point at which the measured values Z_{A} reach local maximum and local minimum from among the time series of the measured values Z_{A}. As shown in Fig. 8, the period determiner 14 then determines the fluctuation period T as the time interval between the earliest extremum time point t₁ after the starting time point to and the extremum time point t₃ that is the next of the next of the extremum time point t₁.
   In the method described in conjunction with Fig. 8, since the fluctuation period T begins at the earliest extremum time point t₁ after starting the measurement of the bioelectrical impedance, the measured values Z_{A} from the starting time point to to the extremum time point t₁ are not used for determining the fluctuation period T and for calculating the determinate value Z_{B}. In contrast to this, since the fluctuation period T begins at the starting time point to in the first embodiment as shown in Fig. 4, the number of the values Z_{A} to be measured by the measuring unit 12 can be reduced as compared to that in this modification described in conjunction with Fig. 8.
   If the length of time of the term including the values Z_{A} to be used for calculating the determinate value Z_{B} is not an integral multiple of the fluctuation period T, the value Z_{B} may vary according to the time position of the term. Since the period determiner 14 in this modification (Fig. 8) accurately determines the fluctuation period T as compared with the first embodiment, the determinate value Z_{B} can be stably calculated regardless of the time position of the values Z_{A} used for calculating the value Z_{B}.
   The fluctuation period T may be set to an integral multiple of one period of the fluctuation in the measured values Z_{A}. In a modification of the third embodiment, the second period determiner 76 may determine the fluctuation period T as a set of two or more candidate periods Tc in the series S.
(2) In the embodiments described above, all of the measured values Z_{A} within the fluctuation period T are used to calculate the determinate value Z_{B} of the bioelectrical impedance. However, the values Z_{A} to be used for calculating the determinate value Z_{B} are not limited to this. For example, only a part of the measured values Z_{A} within the fluctuation period T may be used to calculate the determinate value Z_{B}. More specifically, the calculator 16 may calculate the determinate value Z_{B} as the average of the maximum value and the minimum value of the measured values Z_{A} within the fluctuation period T.
(3) In the embodiments described above, the electrodes (the current supplying electrodes 32 and the voltage measurement electrodes 34) are brought into contact with the abdomen of the human subject. However, the portion of the body to be measured is not limited to the abdomen. For example, the bioelectrical impedance can be measured by using electrodes brought into contact with the hands or the legs of the human subject.
   In an alternative embodiment, the calculation of the value Z_{B} may be repeated with changing the frequency of the measurement current, so that a plurality of the values Z_{B} are obtained at different frequencies. The body composition indexes X may then be calculated on the basis of the plurality of the values Z_{B} (e.g., on the basis of the average of the values Z_{B}).
(4) The index calculator 18 may calculate only one body composition index X. In an alternative embodiment, the index calculator 18 may be omitted. In this embodiment, the output unit 40 outputs the value Z_{B} of the bioelectrical impedance instead of the body composition indexes X.
   The output destination of the body composition index X or the determinate value Z_{B} of the bioelectrical impedance is not limited to the output unit 40. For example, the body composition index X or the determinate value Z_{B} may be stored in the memory 20 or in another memory (e.g., a portable memory). Alternatively, the body composition index X or the determinate value Z_{B} may be sent to another device via a communication network.
(5) In the embodiments described above, the filtering process unit 60 includes the low-pass filter 62 and the high-pass filter 64. However, the configuration of the filtering unit 60 is not limited to this. For example, the filtering process unit 60 may include only one of the low-pass filter 62 and the high-pass filter 64. In an alternative embodiment, a band-pass filter having the pass-band from the cutoff frequency F₁ to the cutoff frequency F₂ may be employed as the filtering process unit 60 instead of the low-pass filter 62 and the high-pass filter 64.

## Claims

1. A bioelectrical impedance measuring apparatus (100, 101) comprising:
a measuring unit (12) for sequentially measuring a bioelectrical impedance of a living subject;
a period determiner (14) for determining a fluctuation period of fluctuation (T) in values of the bioelectrical impedance (Z_{A}) measured by the measuring unit (12), the fluctuation being caused by breathing of the living subject; and
a calculator (16) for calculating an average of a plurality of the values of the bioelectrical impedance (Z_{A}) measured by the measuring unit (12) within the fluctuation period (T) determined by the period determiner (14).

2. The bioelectrical impedance measuring apparatus (100, 101) according to claim 1, wherein
the measuring unit (12) starts measuring the bioelectrical impedance (Z_{A}) at a starting time point (to), the bioelectrical impedance (Z_{A}) reaching one of a local maximum and a local minimum at a first time point (t₁) directly after the starting time point (to), the bioelectrical impedance (Z_{A}) reaching the other of the local maximum and the local minimum at a second time point (t₂) directly after the first time point (t₁),
the period determiner (14) determining the fluctuation period (T) as a sum of twice a time length (T₁) from the starting time point (t₀) to the first time point (t₁) and a time length (T₂) from the first time point (t₁) to the second time point (t₂).

3. The bioelectrical impedance measuring apparatus (100, 101) according to claim 1, wherein
the period determiner (14) determines the fluctuation period (T) as a time interval between two time points at which the bioelectrical impedance reaches two local maximums or minimums.

4. The bioelectrical impedance measuring apparatus (100, 101) according to any one of claims 1 through 3, wherein the calculator (16) calculates the average of all of the values of the bioelectrical impedance (Z_{A}) measured by the measuring unit (12) within the fluctuation period (T) determined by the period determiner (14).

5. The bioelectrical impedance measuring apparatus (100, 101) according to any one of claims 1 through 3, wherein the calculator (16) calculates the average of a maximum value and a minimum value of the bioelectrical impedance (Z_{A}) measured by the measuring unit (12) within the fluctuation period (T) determined by the period determiner (14).

6. The bioelectrical impedance measuring apparatus (101) according to any one of claims 1 through 5, further comprising a low-pass filter (62) for attenuating high-frequency components of the fluctuation in the bioelectrical impedance (Z_{A}) measured by the measuring unit (12), the high-frequency components having frequencies higher than a predetermined cutoff frequency,
wherein the period determiner (14) determines the fluctuation period (T) on the basis of the values of the bioelectrical impedance having passed through the low-pass filter (62),
and wherein the calculator (16) calculates the average of the values of the bioelectrical impedance having passed through the low-pass filter (62).

7. The bioelectrical impedance measuring apparatus (101) according to any one of claims 1 through 6, further comprising a high-pass filter (64) for attenuating low-frequency components of the fluctuation in the bioelectrical impedance (Z_{A}) measured by the measuring unit (12), the low-frequency components having frequencies lower than a predetermined cutoff frequency,
wherein the period determiner (14) determines the fluctuation period (T) on the basis of the values of the bioelectrical impedance having passed through the high-pass filter (64),
and wherein the calculator (16) calculates the average of the value of the bioelectrical impedance having passed through the high-pass filter (62).

8. The bioelectrical impedance measuring apparatus (100, 101) according to any one of claims 1 through 7, wherein the period determiner (14) comprises:
a candidate period determiner (72) for sequentially determining candidate periods of the fluctuation in the values of the bioelectrical impedance (Z_{A}) measured by the measuring unit(12);
a steady period detector (74) for detecting a series of candidate periods each having a time length within a predetermined range; and
a second period determiner (76) for determining the fluctuation period (T) on the basis of the series of the candidate periods detected by the steady period detector (74).

9. A body composition index determining apparatus (100, 101) comprising:
a bioelectrical impedance measuring apparatus (100, 101) according to any one of claims 1 through 8; and
an index calculator (18) for calculating a body composition index of the living subject on the basis of the bioelectrical impedance (Z_{B}) measured by the bioelectrical impedance measuring apparatus (100, 101).
